# EUROPEAN PATENT SPECIFICATION

(11) **EP 0 614 351 B1**
(45) Date of publication and mention of the grant of the patent: **08.04.1998**
(21) Application number: 92925345.8
(22) Date of filing: 25.11.1992
(51) Int. Cl.: A61K 7/08

(54) **SUSPENDING AGENTS FOR INSOLUBLE COMPONENTS OF CLEANING COMPOSITIONS FORMED BY REACTING ALKYL AMMONIUM SALTS WITH ANIONIC SURFACTANTS**
DURCH REAKTION VON ALKYL-AMMONIUMSALZEN MIT ANIONISCHEN TENSIDEN HERGESTELLTE SUSPENDIERMITTEL FüR UNLöSLICHE KOMPONENTE IN REINIGUNGSMITTELN
AGENTS DE SUSPENSION DESTINES A DES ELEMENTS INSOLUBLES DE COMPOSITIONS DE NETTOYAGE, FORMES PAR LA REACTION DE SELS D'AMMONIUM ALKYLE AVEC DES TENSIOACTIFS ANIONIQUES

(30) Priority: 25.11.1991 US 797545
(43) Date of publication of application: 14.09.1994
(73) Proprietor: STEPAN COMPANY, Northfield, Illinois 60093 (US)
(72) Inventor: LEVINSON, Matthew, I., Chicago, IL 60657 (US); SAJIC, Branko, Chicago, IL 60625 (US); DIEZ, Ricardo, Burlington, Ontario L7M 2V6 (CA); BERNHARDT, Randal, J., Lindenhurst, IL 60046 (US)
(74) Representative: Eyles, Christopher Thomas
(86) International application number: US9210016
(87) International publication number: WO9310746

(56) References cited:
- EP-A- 0 166 232
- EP-A- 0 181 773
- EP-A- 0 294 894
- EP-A- 0 313 307
- EP-A- 0 339 539
- EP-A- 0 466 184
- EP-A- 0 467 235
- WO-A-92/10161
- GB-A- 2 196 979
- US-A- 4 954 335
- DATABASE WPI Week 7950, Derwent Publications Ltd., London, GB; AN 79-90068B

## Description

### BACKGROUND OF THE INVENTION

### Field of the Invention

This invention relates to suspending agents for silicone oil conditioning shampoos, which suspending agents are formed by reacting long-chain alkyl ammonium salts with anionic surfactants.

### Description of the Related Art

This prior art is replete with emulsions for a myriad of uses. Among the numerous applications for emulsions are fabric softeners, surface cleaners, agricultural chemical mixtures, skin cleansers, creams and lotions, car wax formulations, textile lubricants, and antiperspirant formulations.

These emulsions are the type wherein a liquid is dispersed in at least one insoluble liquid. Such emulsions include oil-in-water, water-in-oil, oil-in-water-in-a second oil, water-in-oil-in-a second oil, and oil-in-a second oil emulsions. These emulsions typically include oils such as crude petroleum oil, distilled petroleum oil, heavy paraffinic oil, asphaltene oil, linseed oil, tall oil, soybean oil alkyd, linseed oil alkyd, mineral oil, petrolatum, isopropyl palmitate, isopropyl myristate, caprylic/capric triglyceride, lanolin, acetylated lanolin alcohol, silicone compounds such as dimethicone and cyclomethicone, hydrogenated vegetable oil, sesame oil, safflower oil, avocado oil, glycerine, propylene glycol, sorbitol, C₁₂-C₁₆ alcohol benzoates, cocoa butter, vitamin E acetate, squalene, sodium pyrolidone carboxylic acid, methyl glucose ether, panthenol, melanin, and mixtures thereof.

Exemplary emulsions are shampoo and conditioner compositions. The prior art is also replete with shampoo and conditioner emulsions. Such emulsions are widely used because human hair becomes soiled due to its contact with the surrounding atmosphere and, to a greater extent, from sebum secreted by the head. The build-up of the sebum causes the hair to have a dirty feel and an unattractive appearance. The soiling of the hair necessitates its being shampooed with frequent regularity.

Shampooing the hair cleans by removing soil and sebum. However, the shampooing process is disadvantageous because the process results in hair that is left in a wet, tangled and generally unmanageable state. A variety of approaches have been developed to alleviate these after-shampoo problems. These approaches range from the inclusion of hair conditioning aids in shampoos to post-shampoo application of hair conditioners, i.e., hair rinses. Post shampoo hair rinses or conditioners typically work by depositing a polymeric film or other material onto the hair. However, the use of solutions of such material as conditioners has not been fully satisfactory. For one thing, hair rinses are generally liquid in nature and must be applied in a separate step following the shampooing, left on the hair for a length of time, and finally rinsed with fresh water. This, of course, is time consuming and inconvenient. Furthermore, hair rinses or the leave-on hair conditioners, in addition to requiring an extra step, are difficult to apply in just the right amount of product and cannot easily be distributed uniformly throughout a head of hair.

Shampoos have been disclosed which contain conditioning aids; however, these shampoos have not been totally satisfactory for a variety of reasons. One reason relates to a lack of compatibility between surfactants which are good cleaning agents and fatty cationic agents which are good conditioning agents. This lack of compatibility caused other surfactants such as nonionics, amphoterics and zwitterionics to be examined by workers in the field.

Suspending/emulsifying agents have been used for cationics in shampoo compositions with surfactants and silicone materials. Normally, a suspension system comprising of xanthum gum, glycerol distearate and cetyl alcohol is used. Manufacturing these compositions is extremely complex, costly and time consuming.

The use of silicone material in shampoos has been described in a number of different publications. The manufacture of such compositions is extremely complicated and requires specialized mixing equipment, high shear pumps, a heat exchanger, several manufacturing tanks, etc. Although it is desirable to reduce the amounts of silicone compounds in cleaning compositions, little success has been evident in this area.

U.S. Patent 4,741,855 describes shampoo compositions which comprise a synthetic surfactant, an insoluble, non-volatile silicone, a suspending agent, and water. The described suspending agents include long chain esters of ethylene glycol, esters of long chain fatty amine oxides and many others. There appear to be several key conditioning components in these compositions, including an insoluble, non-volatile, silicone, and a long chain acyl suspending agent such as an ethylene glycol ester of a fatty acid, an alkanolamide, or an alkyl dimethyl amine oxide. Optional components that may be included in these compositions may include cationic surfactants such as di(partially hydrogenated) tallow dimethyl ammonium chloride and cetyltrimethyl ammonium chloride.

European patent application No. 89312074.1 teaches hair care compositions comprising from about 0.05% to about 10.0% of a nonrigid silicone gum. Dispersed in the gum is from about 0.01% to about 8.0% of an unsolubilized particulate matter which is preferably an octylacrylamide/acrylate/butylaminoethyl methacrylate copolymer. In addition, these compositions include volatile silicone solvents, silicone resins and optional ingredients. Among the optional ingredients are cationic surfactants of the formula 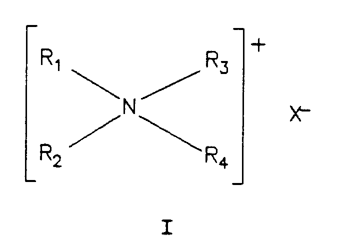 wherein R₁ is hydrogen, an aliphatic group of from 1-22 carbon atoms, or an aromatic, aryl or aralkyl group having from 12 to 22 carbon atoms, R₂ is an aliphatic group having from 1 to 3 carbon atoms, and X is an anion selected from halogen, acetate, phosphate, nitrate and alkylsulfate radicals.

Quaternary ammonium compounds derived from fatty acid amines such as tallow amine and di-tallow amine have been used as conditioners, surfactants and thickeners or emulsifiers in various shampoo and hair care products. For example, European Patent Application No. 0067635A2 discloses conditioning shampoos containing quaternary ammonium compounds of the formula: 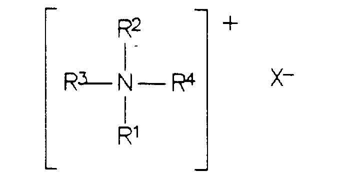 wherein the R¹ and R² groups contain an average of from about 16 to 22 carbon atoms, most preferably from about 16 to about 18 carbon atoms, R³ and R⁴ are C₁ to C₄ alkyl or hydroxyalkyl groups, and X is any compatible anion, particularly one selected from the group consisting of halide, hydroxide, methylsulfate, or acetate anions.

The shampoo compositions of that patent application also contain acyl derivatives which are long chain amides, alkanolamides, esters of ethylene glycol and glycerine, esters of carboxylic acids, esters of thiodicarboxylic acids, and mixtures of these derivatives. The shampoo compositions of that patent application also contain surfactants which are represented by the formula: 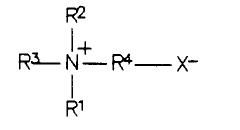 wherein R¹ is a long chain alkyl radical having from about 10 to about 18 carbon atoms or an amido radical represented by the formula: 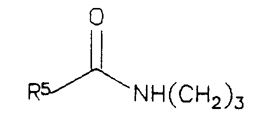 wherein R⁵ is a long chain alkyl radical, R² and R³ are each alkyl radicals having from about 1 to about 3 carbon atoms, R⁴ is an alkylene or hydroxy alkylene radical having from about 1 to about 4 carbon atoms, and X is a carboxylate radical.

European Patent Application No. 0 152 194 A2 discloses shampoo compositions containing aminofunctional silicones and quaternary ammonium salts of the formula: 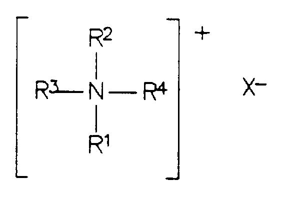 wherein R₁ is hydrogen, or an aliphatic group of from 1 to 22 carbon atoms, or an aromatic, aryl or alkaryl group having 6 to 20 carbon atoms; R₂ is an aliphatic group having from 12 to 22 carbon atoms; R₃ and R₄ are each alkyl groups having from 1 to 3 carbon atoms; and X is an anion selected from halogen, acetate, phosphate, nitrate and methyl sulfate radicals.

U.S. Patent No. 4,854,333 discloses shampoo compositions that may contain di(partially hydrogenated tallow) dimethyl ammonium chloride, an alkyl sulfate and a silicone fluid. The alkyl sulfates include the sodium and ammonium alkyl sulfates having 8-22 carbon atoms.

U.S. Patent No. 4,824,602 discloses hair care compositions comprising silicone conditioning agents and compounds of the following formula: 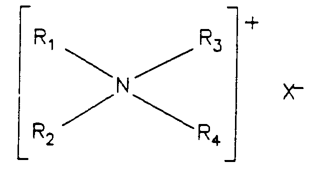 wherein R₁ and R₂ are aliphatic groups having from 12-22 carbon atoms, R₃ and R₄ may be hydrogen or short chain alkyl groups, and X may be an alkyl sulfate radical.

The preferred quaternary ammonium salts according to this patent are dialkyl dimethyl ammonium chlorides. The alkyl groups include those derived from long-chain fatty acids such as hydrogenated tallow fatty acids. These shampoos may also include anionic surfactants which may be alkyl sulfates having between 10 and 20 carbon atoms where the cation may be ammonium.

U.S. Patent No. 4,788,006 teaches shampoo compositions that include alkyl sulfates such as ammonium lauryl sulfate, a silicone fluid, and, as an optional component, a quaternary ammonium compound such as di(partially hydrogenated tallow) dimethyl ammonium chloride.

U.K. Patent Application No. GB 2 196 979A discloses hair care compositions comprising silicone conditioning agents and compounds of the following formula: 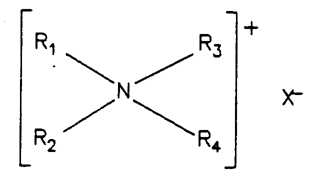 wherein R₁ and R₂ are aliphatic groups having from 12-22 carbon atoms, R₃ and R₄ may be hydrogen or short chain alkyl groups, and X may be an alkyl sulfate radical.

The preferred quaternary ammonium salts according to this patent are dialkyl dimethyl ammonium chlorides. The alkyl groups include those derived from long-chain fatty acids such as hydrogenated tallow fatty acids. These shampoos may also include anionic surfactants which may be alkyl sulfates having between 10 and 20 carbon atoms where the cation may be ammonium.

U.K. Patent Application No. GB 2 196 980A discloses hair care compositions comprising silicone conditioning agents and compounds of the following formula: 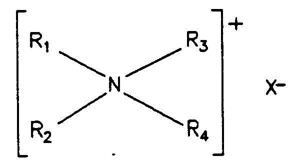 wherein R₁ and R₂ are aliphatic groups having from 12-22 carbon atoms, R₃ and R₄ may be hydrogen or short chain alkyl groups, and X may be an alkyl sulfate radical.

The preferred quarternary ammonium salts according to this patent are dialkyl dimethyl ammonium chlorides. The alkyl groups include those derived from long-chain fatty acids such as hydrogenated tallow fatty acids. These shampoos may also include anionic surfactants which may be alkyl sulfates having between 10 and 20 carbon atoms where the cation may be ammonium.

U.K. Patent Application No. GB 2124647 A teaches quaternary ammonium compounds useful in shampoo compositions. The ammonium compounds have the formula: 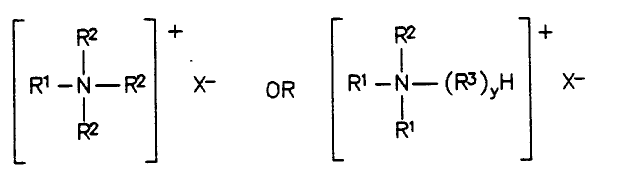 wherein R₁ is an aliphatic alkyl group containing an average of from about 16 to 22 carbon atoms, most preferably from about 16 to about 18 carbon atoms, the R₂ groups are C₁ to C₄ alkyl or hydroxylalkyl groups, the R₃ groups are alkylene oxide groups, preferably propylene oxide, where y is 1-4 and X is any compatible anion, particularly one selected from the group consisting of halide, hydroxide, methylsulfate, or acetate anions.

European Patent Application No. 0294 894 discloses conditioning agents for delivery from shampoos comprising compounds of the formula: 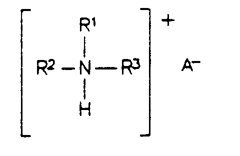 wherein R₁ and R₂ can independently be C₁₆ to C₂₀ alkyl or alkenyl and R³ is H or CH₃, and A is an anionic surfactant selected from the group consisting of alkyl sulfonates, aryl sulfonates, alkylaryl sulfonates, alkyl sulfates, alkyl ethoxylated sulfates, dialkyl sulfosuccinates, ethoxylated alkyl sulfonates, alkyl oxybenzene sulfonates, acyl isethionates, acyl alkyl taurates, olefin sulfonates and paraffin sulfonates.

A wide variety of surface active (surfactant) compounds are known and widely used. Further, certain relatively specific phthalamate derivatives are at least nominally disclosed in academic and patent literature. Some specific phthalamate derivatives have been suggested as being useful in plant growth regulator formulations, insect repellent formulations, bactericidal, fungicidal, or herbicidal formulations, additives for improving low temperature flow characteristics of petroleum distillate fuels, solvent extraction formulations for certain heavy metal ions, catalyst systems for polyurethane foam formulations, additives for thermal recording materials, thickeners for silicone grease and oil-based drilling muds, additives for water-insensitive coatings, and plasticizers. Phthalamic acids or phthalamate derivatives have been used as additives for insecticidal compositions; additives for vulcanization activators; additives for rust and corrosion inhibitor formulations; additives to screen-clogging prevention and rust inhibition formulations; additives for improving low temperature flow characteristics of petroleum fuel oils; and additives to catalyst systems for polyurethane foam formulations.

Ammonium phthalamates have been used as additives in fuel oil compositions, blending agents for grease, lubricating oil additives, and thickening agents for lubricating oil compositions.

These ammonium phthalamates have the formula 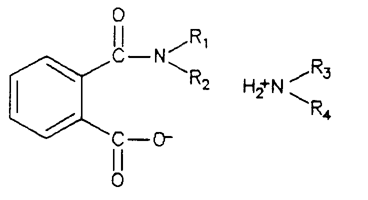 wherein
R₁, R₂, R₃ and R₄ are the C₁₆-C₄₀, preferably C₁₆-C₂₄ straight chain alkyl groups of secondary amine, and may be the same or different.

U.S. Patent No. 5,015,415 discloses formulated conditioning shampoos, surfactant solutions and emulsifier solutions comprising effective amounts of salts of the general formula 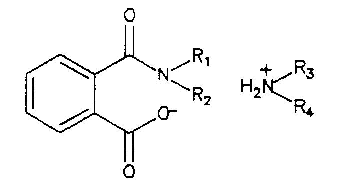 and mixtures of the salts and acids of the formula

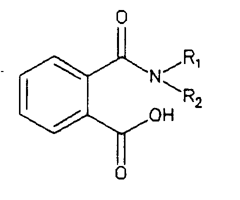

U.S. Patent No. 2,101,323 discloses compounds of the formula 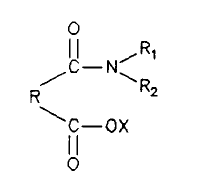 in which X is hydrogen or an alkali or alkaline earth metal, R represents the residue of a dicarboxylic acid, preferably an ortho-aromatic radical of the benzene series, R₁ is an aliphatic hydrocarbon radical containing eight or more carbon atoms and R₂ is hydrogen or anorganic radical, and which may be used as water-resistant, low-vapor pressure plasticizers for cellulose derivatives, and as wetting and detergent agents.

U.S. Patent No. 2,892,778 teaches grease compositions comprising metal soaps of terephthalic acid, terephthalamic acid, isophthalamic acid, and amic acids. U.S. Patent No. 2,0915,464 teaches hydrocarbon lubricating oils comprising salts N-alkylamidophthalic acids where the N-alkyl substituent contains 8 to 18 carbon atoms.

U.S. Patent No. 2,971,027 discloses the use of diamides of terephthalic acid as grease thickening agents. These compounds have the general formula 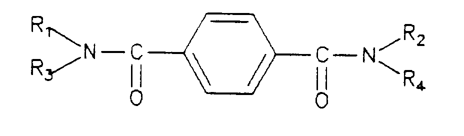 where R₁ and R₂ are selected from the group consisting of hydrocarbon and substituted hydrocarbon radicals attached to the nitrogen atoms by carbon-nitrogen bonds, and wherein R₃ and R₄ are selected from the group consisting of hydrogen, hydrocarbon and substituted hydrocarbon radicals, such hydrocarbon or substituted hydrocarbon radicals being attached to the nitrogen atoms by carbon-nitrogen bonds.

U.S. Patent No. 3,095,286 discloses compounds of the general formula 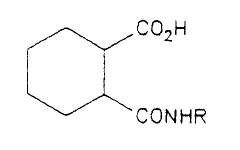 where R is a monovalent aliphatic hydrocarbon radical having between about 4 and 30 carbon atoms as being useful as fuel oil sediment inhibitors, anti-rust agents, and anti-screen clogging agents.

U.S. Patent No. 3,166,387 teaches pour point depressors for fuel oil compositions comprising salts of a secondary or tertiary monoamine having the formula: 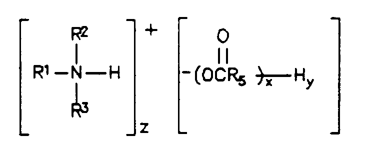 where R₁ and R₂ are alkyl groups containing from about 10 to about 22 carbon atoms, and R₃ and R₅ are hydrogen or alkyl groups containing from one to about 22 carbon atoms, x and z are each integers of from 1-4 and y is an integer of from 0 to 1.

U.S. Patent Nos. 4,333,082 and 3,544,467 disclose pour point depressants for hydrocarbon fuels and oils comprising succinamic acids of the formula 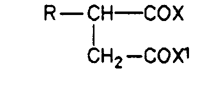 wherein R is a straight chain aliphatic hydrocarbon group having from 0 to 1 site of olefinic unsaturation (alkyl or alkenyl) attached at a secondary carbon atom to the succinyl group and is of at least 14 carbon atoms, generally in the range of 15 to 28 carbon atoms and more usually in the range of 15 to 22 carbon atoms. One of X and X¹ is hydroxyl and the other is:

-NYY¹

wherein N has its normal meaning of nitrogen and Y and Y¹ are aliphatic hydrocarbyl groups of from 14 to 28 carbon atoms, more usually of from 15 to 22 carbon atoms, having a total of from about 30 to 52 carbon atoms, more usually of from 32 to 48 carbon atoms, and, preferably, of from 32 to 40 carbon atoms, and the salts thereof.

U.S. Patent No. 3,658,453 teaches the use of various limited solubility wax crystal modifying amides or salts formed from acids with amines or ammonia to improve the cold flow properties of distillate fuel oils. Among the salts disclosed are various ammonium salts of fatty acids.

U.S. Patent No. 3,846,481 discloses di-(n-octadecyl) ammonium salts of aromatic carboxylic acids and their use as pour point depressants in hydrocarbon oils.

U.S. Patent No. 3,887,754 teaches the use of phthalic acid derivatives of the formula 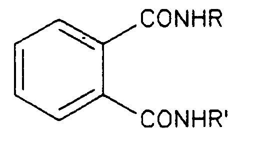 where R and R¹ are aliphatic hydrocarbon radicals of from 8 to 22 carbon atoms as coating substances for foamed polystyrene articles.

U.S. Patent No. 3,982,909 describes the use of various amides, diamides and ammonium salts of monoamides or monoesters of dibasic acids as wax crystal modifiers and cold flow improves for distillate fuel oils.

U.S. Patent Nos. 4,210,424 and 4,211,534 teach the case of oil-soluble combinations of (A) ethylene polymer of copolymer, (B) normal paraffinic wax composed of normal hydrocarbons whose average molecular weight is within the range of from 300 to 650, and (C) nitrogen compounds, such as amides, amine salts and ammonium salts, of carboxylic acids or anhydrides for use in improving the cold flow properties of distillate hydrocarbon fuel oils.

Journal of Colloid and Interface Science, 139: 381 (1990) describes the use of compounds of the formula 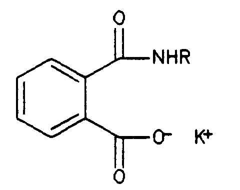 where R is octyl, dodecyl or oleyl as surfactants which stabilize water-in-oil microemulsions.

Tallow is a fatty acid byproduct of the meat-packing industry obtained by rendering the body fat from cattle and sheep. Tallows from different sources vary in free fatty acid content. The fatty acids normally found in tallow are myristic acid, palmitic acid, stearic acid, oleic acid, and linoleic acid.

Several methods are known for the preparation of tallow amines, but the most common method in industry is the conversion of a fatty acid to a nitrile by treating with ammonia, followed by catalytic hydrogenation of the nitrile to primary, secondary, or tertiary amine by suitable adjustment in the reaction conditions. Tallow amines, as well as di(hydrogenated tallow) amine, are commercially available; for example, di(hydrogenated tallow) amines are available under the trade name ARMEEN 2HT™ (Akzo Chemicals, Chicago, Illinois).

Various routes exist for the preparation of phthalamic acids and phthalamic acid salts. In U.S. Patent No. 4,402,708 N,N-diarachidyl phthalamic acid was prepared by adding phthalic anhydride to a 40% solution of amine in toluene in a 1/1 mole ratio at 80°C. The product was recovered by vacuum drying at 50°C, 0.05 mmHg for 20.5 hours. Phthalic anhydride sublimation was observed. This method makes no mention of the presence of any ammonium salt in the resultant product.

U.S. Patent No. 4,402,708 describes a method for preparing N,N-dioctadecyl phthalamic acid dioctadecyl ammonium salt and N,N-diarachidyl phthalamic acid diarachidyl ammonium salt. Phthalic anhydride was added to a 10% solution of amine in toluene in an anhydride to amine mole ratio of 1/2. The product was recovered by filtering and film evaporating a 1/1 toluene/n-heptane solution at 55°C, 40 mmHg.

Phthalamic acids have also been prepared by melting phthalic anhydride at 131°C and subsequent addition of molten secondary amine. The reactants to be added in an equimolar ratio. At the temperature used in this method, 131°C, excessive phthalic anhydride sublimation occurs and increased product degradation is observed. This method makes no mention of the presence of any ammonium salt in the resultant product.

Phthalamic acids have been prepared by addition of a solution of secondary amine in isopropanol at 78°C to a phthalic anhydride/isopropanol slurry at 78°C in a one to one phthalic anhydride/amine molar ratio with subsequent vacuum stripping of the solvent. This method utilizes isopropanol as the solvent for the reaction. Isopropanol, a secondary alcohol, reacts with phthalic anhydride to yield isopropyl mono ester of phthalic acid. At 78°C, as much as 40% of the product may be this ester.

Each of these methods produces a mixture of the desired phthalamic acid and an ammonium phthalamate salt. However, these methods make no mention of the presence of any ammonium salt in the resultant product.

### Summary of the Invention

It has been unexpectedly discovered that when a di-(C₈ to C₄₀ alkyl) ammonium salt and a silicone oil are added to a liquid shampoo composition containing an anionic alkyl sulfate surfactant and the mixture heated above 70°C, the resulting composition contains a product which functions as an excellent suspending agent for insoluble components of the liquid shampoo composition. These suspending agents appear as particles that have precipitated in the shampoo composition.

Accordingly, the present invention provides suspending agents for insoluble components of liquid shampoo compositions that are prepared by a process comprising the steps of (a) mixing a di-(C₈ to C₄₀ alkyl) ammonium salt, a silicone oil and an anionic surfactant in the shampoo composition; and (b) heating the mixture above 70°C.

In addition, the present invention provides suspending agents for silicone oil conditioning agents of shampoos that are prepared by a process comprising the steps of (a) mixing a di(hydrogenated) tallow ammonium salt and a salt of lauryl sulfate in the shampoo; and (b) heating the mixture to at least 70°C.

The invention also provides liquid cleaning compositions comprising effective amounts of such suspending agents for suspending insoluble components of liquid shampoo compositions.

The invention further provides networks of the suspending agents and the insoluble components of liquid shampoo compositions.

The invention also provides shampoo compositions containing the suspending agents.

### BRIEF DESCRIPTION OF THE DRAWING

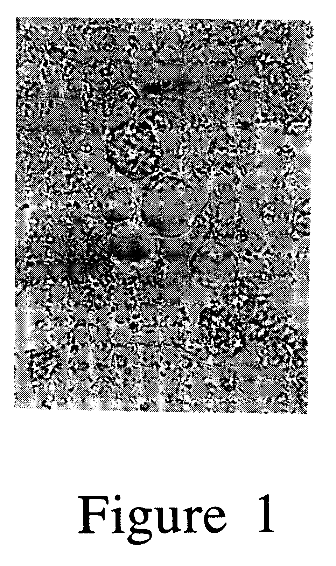

Figure 1 is a photograph at a magnification of 125X showing spherical droplets of silicone oil conditioning agent (A) adhered to particles of suspending agent (B).

### DETAILED DESCRIPTION OF THE INVENTION

It has been unexpectantly discovered that when a di-(C₈ to C₄₀ alkyl) ammonium salt is added to a liquid shampoo composition containing an anionic surfactant and a silicone oil and the mixture is heated above 70°C, the resulting composition contains a product which functions as an excellent suspending agent for insoluble components of the liquid shampoo composition. These suspending agents appear as particles that have precipitated in the insoluble components of the liquid shampoo composition.

Thus the present invention encompasses suspending agents for insoluble components of liquid shampoo compositions, which suspending agents are prepared by a process comprising the steps of (a) mixing a di-(C₈ to C₄₀ alkyl) ammonium salt, a silicone oil and an anionic surfactant in the shampoo composition; and (b) heating the mixture above 70°C.

The present invention further encompasses suspending agents for insoluble components of liquid shampoo compositions, which suspending agents are prepared by a process comprising the steps of (a) mixing a di(hydrogenated) tallow ammonium salt, a silicone oil and a salt of lauryl sulfate in the shampoo composition; and (b) heating the mixture above 70°C.

The invention also encompasses liquid shampoo compositions formulated to include an effective amount of such suspending agents for suspending insoluble components of liquid shampoo compositions.

Thus, in accordance with one aspect of the invention there is provided a method of making a shampoo comprising an alkyl sulfate surfactant, a silicone conditioning agent and a quaternary ammonium salt characterized in that it includes the step of forming a composition of silicone adhered to particles of a suspending agent by heating to a temperature of at least 70°C
(a) a shampoo base comprising an alkyl sulfate surfactant of the formula: 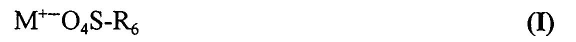 where
   R₆ is alkyl having from 10 to 16 carbon atoms; and
   M is sodium, potassium, or NRR₇R₈R₉R₁₀
   where R_{,7,} R_{8,} R_{9,} and R₁₀ are the same or different and represent hydrogen or straight or branched chain alkyl groups having 1 to 8 carbon atoms;
(b) a silicone oil; and
(c) a di-(C₈ to C₄₀ alkyl) quaternary ammonium salt of the formula: 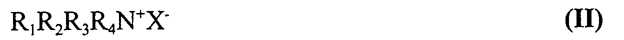 where R₁ and R₂ are the same or different and represent alkyl having 8 to 40 carbon atoms;
   R₃ and R₄ are hydrogen; and
   X is halide, acetate, nitrate, hydroxide, phosphate;
   alkyl sulfate where the alkyl portion has 1-22 carbon atoms;
   mono- or di-alkyl phosphate where each alkyl portion has 1-22 carbon atoms;
   an anion of the formula

      R₅-CO⁻₂
   where R₅ is straight or branched chain alkyl having 1-22 carbon atoms; or 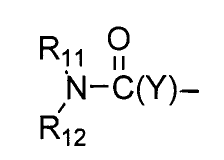 where R₁₁ and R₁₂ are the same or different and represent alkyl or alkenyl having 1 to 40 carbon atoms; and
   Y is ortho, para or meta substituted phenylene, or alkylene or alkenylene having 0-6 carbon atoms, or cycloalkylene having 5-7 carbon atoms.

This invention further provides a composition comprising a network of silicone oil conditioning agent droplets adhered to a suspending agent which is the reaction product of a di- (C₈ ro C₄₀ alkyl) quaternary ammonium salt of the formula (II) set out abovr and an alkyl sulfate surfactant of the formula (I) set out above.

Also provided in accordance with the invention is a shampoo comprising an alkyl sulfate surfactant and a network of an effective conditioning amount of silicone conditioning agent droplets adhered to a suspending agent which is the reaction product of a di-(C₈ to C₄₀ alkyl) quaternary ammonium salt of the formula (II) set out above and an alkyl sulfate surfactant of the formula (I) set out above.

In a further aspect of the present invention there is provided a conditioning shampoo comprising a silicone oil conditioning agent stably suspended in a network of silicone oil adhered to a suspending agent, the shampoo being prepared by a process comprising the steps of (a) mixing an alkyl ammonium salt of the formula R₁R₂NH₂⁺X⁻, where X is as defined above and R₁ and R₂ are the same or different and represent straight or branched alkyl groups having from 14 to 20 carbon atoms, and an excess of an alkyl sulfate surfactant in the shampoo, the alkyl sulfate surfactant being of the formula:

M⁺⁻O₄S-R₆ (I)

where M and R₆ are as defined above; and (b) heating the shampoo above 70°C.

By alkyl in the present invention is meant straight or branched chain alkyl groups. By alkenyl is meant straight or branched chain alkenyl groups having at least one carbon-carbon double bond. When the alkenyl groups have a plurality of double bonds, the double bonds may be conjugated or not conjugated.

The preferred alkyl ammonium salts for utilization in the present invention are di(hydrogenated) tallow ammonium salts. These di(hydrogenated) tallow ammonium salts may be represented by the formula:

R₁R₂NH₂⁺X⁻

where R₁ and R₂ are the same or different and represent straight or branched chain alkyl groups having 14-20, preferably 16-20, carbon atoms; and
X is as defined above.

By Y in the above formulas is meant an ortho, meta, or para substituted phenylene group, or methylene, ethylene, propylene, butylene, pentylene, hexylene, ethenylene, propenylene, 1-, or 2-butenylene, 1-, 2-, or 3-pentylene, or 1-, 2-, or 3-hexenylene, cyclohexylene or cyclohexenylene. The amic acid anions resulting from the variation of y groups include phthalamate, isophthalamate, terephthalamate, oxalamate, malonamate, glutaramate, adipamate, pimelamate, suberamate, maleamate, succinamate, 1,2-cyclohexanamate, 1,3-cyclohexanamate, and 1,4 cyclohexanamate anions.

Mixtures of various di-(C₈ to C₄₀ alkyl) ammonium salts and anionic surfactants may be utilized in the present invention to achieve different suspending properties as desired.

Suitable di(hydrogenated) tallow ammonium salts are easily prepared and/or commercially available. The alkyl ammonium salts derived from tallow amine include various amic acid ammonium salts. In certain embodiments of the invention, R₁, R₂, R₃, and R₄, are derived from hydrogenated tallow. Because tallow is derived from a mixture of C₁₄ to C₁₈ fatty acids, and amines derived from tallow are a mixture of tallow amines, the amic acid ammonium salts thereof used in the present invention may, therefore, have R groups that are the same or different. Preferred amic acid ammonium salts according to the present invention include, for example, di(hydrogenated) tallow ammonium di(hydrogenated) tallow succinamate, di(hydrogenated) tallow ammonium di(hydrogenated) tallow cyclohexamate, di(hydrogenated) tallow ammonium di(hydrogenated) isophthalamate, di(hydrogenated) tallow ammonium di(hydrogenated) tallow maleamate, di(hydrogenated) tallow ammonium di(hydrogenated) tallow oxalamate, di(hydrogenated) tallow ammonium di(hydrogenated) tallow malonamate, di(hydrogenated) tallow ammonium di(hydrogenated) tallow glutaramate, di(hydrogenated) tallow ammonium di(hydrogenated) tallow adipamate, di(hydrogenated) tallow ammonium di(hydrogenated) tallow pimelamate, and di(hydrogenated) tallow ammonium di(hydrogenated) tallow subaramate. A particularly preferred alkyl ammonium salt is di(hydrogenated) tallow ammonium di(hydrogenated) tallow phthalamate.

Similarly, anionic surfactants are easily prepared and/or commercially available. Among the anionic surfactants suitable for use in the present invention are: alkyl polyalkoxy sulfates and sulfonates; aralkyl sulfates, sulfonates, and phosphates; alkylaryl sulfates and sulfonates; α-olefin sulfates and sulfonates; α-sulfo-alkyl alkanoates; and alkyl sulfates, sulfonates and phosphates.

The preferred anionic surfactants that may be used in the invention are salts of lauryl sulfate. These salts of lauryl sulfate may be represented by the formula

M⁺⁻O₄S-R₆

where R₆ is an alkyl group having from 10-16 carbon atoms; and
M is sodium, potassium, or NR₇R₈R₉R₁₀ where R₇, R₈, R₉ and R₁₀ are the same or different and represent hydrogen or straight or branched chain alkyl groups having 1-8 carbon atoms.
A particularly preferred salt of lauryl sulfate is ammonium lauryl sulfate.

Suitable amounts of di-(C₈ to C₄₀ alkyl) ammonium salt are from about 0.5% to about 20% of a cleaning composition. Preferred amounts are from about 2-10%. More preferred amounts of the di-(C₈ to C₄₀ alkyl) ammonium salts are from about 3-7%.

The anionic surfactant may be used in an amount in excess of the di-(C₈ to C₄₀ alkyl) ammonium salt. Suitable amounts of anionic surfactants are from about 1% to about 50% of a cleaning composition. Preferred amounts are from about 5-30%. More preferred amounts of anionic surfactants are from about 8-20% of a cleaning composition.

After preparation of a formulated shampoo according to the invention, the preparation was filtered, and particles collected. Chemical analysis of the particles indicated the particles are the product of a reaction between the cationic portion of the alkyl ammonium salt and the anionic portion of the surfactant.

Upon preparation of a formulated conditioning shampoo including a silicone oil conditioning agent and a suspending agent according to the invention, the preparation was filtered, and particles collected. Microscopic analysis of these particles reveals that the silicone oil conditioning agent and suspending agent particles are a network of the silicone oil adhered to the particles of the suspending agent. Figure 1 shows the larger silicone oil droplets (A) adhered to the smaller particles (B) of a suspending agent prepared from a tallow ammonium compound and lauryl sulfate. The network effectively maintains silicone conditioning agents in suspension in cleaning compositions such as shampoo formulations.

By network is meant an arrangement or complex of silicone oil conditioning agent droplets adhered to suspending agent particles to form an interconnected system of silicone and suspending agent.

The compositions and shampoos of the invention may be prepared to comprise from about 0.05% to 35% by weight of the network formed by silicone oil droplets adhered to particles of the suspending agent. Preferred amounts of the network are from about 2-30% by weight of the composition. More preferred amounts are from about 3-25% by weight of the compostion.

The silicone compounds which may be used in the formulations of the present invention are well known and have been generally taught to be useful in a variety of emulsions, suspensions, and non-aqueous systems. These silicone compounds include silicones such as dimethicone and cyclomethicone. Generally such silicone compounds may be represented by the formula: 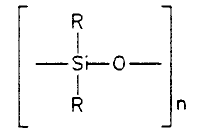 wherein R is a 1 to 3 carbon alkyl group, n is a number from 3 to 10, preferably from 3 to 7, and the unsatisfied valences on the oxygen and silicon atoms at the ends of the chain may be joined to one another to form a cyclic structure. Suitable silicone compounds are, for example, U.C.C. Y-7207, sold by Union Carbide Corporation in which each R is methyl and which typically comprises by weight 99.4% tetramer, 0.6% trimer and traces of the pentamer and hexamer; SWS-03314, sold by SWS Silicones, a Division of Stauffer Chemical Company, in which R is methyl and which is substantially all tetramer; and Dow Corning 344 fluid, sold by Dow Corning, Inc., in which R is methyl and which typically comprises by weight about 88% tetramer, about 11.8% pentamer and traces of trimer and hexamer. Typical vapor pressures of silicones are shown below. These vapor pressures were determined using Dow Corning 344 fluid at various temperatures.

| Temperature | Vapor Pressure, mm Hg (Pa) |
|---|---|
| 26°C | 1 (133.3) |
| 64°C | 10 (1333.2) |
| 77°C | 20 (2666.4) |
| 92°C | 40 (5332.9) |
| 101°C | 60 (7999.3) |
| 114°C | 100 (13332.2) |
| 155°C | 400 (533329.0) |
| 178°C | 760 (101325.0) |

The concentration of silicone oil conditioning compound in the formulations and shampoos of the invention may range from about 0.05% to about 15% by weight of the composition. More preferred amounts are from about 0.5 to 10% of the formulation.

By suspension is meant a mixture of particles or solid particulate of matter or droplets of an insoluble liquid evenly distributed throughout a liquid system in which the particles or droplets are insoluble. The particles or droplets may be of any composition, i.e., they may be organic or inorganic compounds. In addition, various organometallic compounds, such as the solid, insoluble, active antiperspirant aluminum and zirconium salts, may be included in the formulations of the invention. These aluminum and zirconium salts include aluminum chloride, aluminum chlorhydroxide, basic aluminum bromide, zirconyl chloride, zirconyl hydroxide, complexes of aluminum hydroxide, zirconyl chloride and aluminum chlorhydroxide, complexes of aluminum hydroxide, zirconyl hydroxychloride and aluminum chlorhydroxide, complexes of dihydroxyaluminum glycinate, zirconyl chloride and/or zirconyl hydroxychloride and aluminum chlorhydroxides, complexes of zirconyl chloride and/or zirconyl hydroxychloride with aluminum chlorhydroxide and an amino acid, such as glycine (as a buffering agent). The liquid systems may be aqueous, nonaqueous, or an emulsion as described below.

By emulsion is meant a mixture of the type wherein a liquid is dispersed in at least one insoluble liquid. Those skilled in the art will recognize a variety of emulsions where the compounds of the present invention may be used. The emulsions of the invention include emulsions such as, for example, oil-in-water, water-in-oil, oil-in-water-in-a second oil, water-in-oil-in-a-second oil, and oil-in-a second oil emulsions.

The shampoos of the present invention are readily manufactured using a conventional single-phase, hot process. The manufacture of the conditioning shampoos using a single-phase, hot process is simple and, therefore, preferred over multi-phase processes.

The shampoos of the present invention are prepared by incorporating the di-alkyl ammonium salt and anionic surfactant in typical shampoo bases. The shampoo formulations of the invention may also include pearling agents, emollients, humectants, proteins, amino acids, and polymers. Those skilled in the art will recognize that desired properties may be attained by adding certain ingredients to these formulations. A representative list of such ingredients may be found in the Cosmetic, Toiletry and Fragrance Association (CTFA) Dictionary. For example, thickeners and viscosity modifiers such as a diethanolamide of a long-chain fatty acid (e.g., cocomide diethanolamine) may be added to the formulations of the present invention.

Because tallow is a mixture of fatty acids of various chain lengths, predominantly C₁₄ to C₁₈, and amines derived from tallow are a mixture of tallow amines, the di-(hydrogenated) tallow ammonium salts preferably used in the present invention may, therefore, have alkyl groups that are the same or different.

Laboratory and salon results indicate that the suspending agents for silicone oil conditioning compounds as prepared according to the invention, when combined with silicone oils, exhibit excellent flow properties and long term stability at a variety of storage temperatures. The finished formulations retain their conditioning, flow and storage properties after at least three freeze-thaw cycle and are easily preserved using common preservatives.

The present invention also encompasses methods for preparing the suspending agents of the invention.

The invention is illustrated further by the following examples which are not to be construed as limiting the invention in scope or spirit to the specific procedures described in them.

### EXAMPLE 1

### Preparation of a mixture of N,N-di(hydrogenated tallow) phthalamic acid and N,N-di(hydrogenated tallow) phthalamic acid di-(hydrogenated tallow) ammonium salt

One mole (148.0g) of flaked phthalic anhydride (PA) was charged into a 4-neck 5-liter round bottomed flask reactor equipped with a mechanical stirrer, a thermocouple temperature controller and a heating mantle. A charge of 650g of isopropyl alcohol (USP grade) was added to the reactor to achieve a slurry of about 50% solids. One mole (about 502g) of molten di(hydrogenated tallow) amine was slowly added to the slurry in the reactor with continuous stirring. The temperature of the reaction mass was allowed to stabilize at about 45-55°C with gradual addition of the amine and cooling of the reactor flask. Amine addition was completed in about 0.5 to 1 hours. Thereafter the reactor was maintained at about 60°C until the PA flakes dissipated and IR spectroscopic analysis showed no detectable amounts of PA in the reaction mass. Isopropyl alcohol was then removed under vacuum (about 1-50 mmHg) On analysis, about 62 mole % acid and about 38 mole % salt were found with an average molecular weight of about 782.

### EXAMPLE 2

Maleic anhydride (0.25 mol) was added to a flask and heated to about 80°C. To the liquid maleic anhydride was added 0.338 mol of liquid di(hydrogenated) tallow amine. The resulting mixture was stirred for 150 minutes at 80°C and, after workup, yielded a mixture of di(hydrogenated) tallow ammonium di(hydrogenated) tallow maleamate and di(hydrogenated) tallow maleamic acid.

### EXAMPLE 3

### Preparation of a Conditioning Shampoo

Water (70.0g) was first added into a suitable vessel equipped with agitation, heating and cooling capabilities. While the water was agitated and heated slowly, tetrasodium EDTA (0.2g), STEPANOL® AM-V (ammonium lauryl sulfate 2g) and NINOL® 40 CO (cocomide diethanolamine 2g) were added. At about 60°C a 70:30 mixture of N, N-di(hydrogenated tallow) phthalamic acid and N,N-di(hydrogenated tallow) phthalamic acid N,N-di(hydrogenated tallow) ammonium salt (5g), followed by silicone DC 200 (dimethicone (2g)) were added to the mixture. The mixture was heated to 70-75°C and emulsified for 20-30 minutes at high speed while maintaining the temperature between 70-75°C. The mixture was slowly cooled with agitation set at medium speed. When the temperature of the mixture cooled to about 45°C, 1,3,5,5-tetramethyl hydantoin (0.1g) and ammonium chloride (0.1g) were added. The pH was checked and adjusted as necessary with ammonium hydroxide or citric acid to a value between about 4.5 to 6.5. The viscosity was checked and adjusted as necessary with ammonium chloride to a value between 2000 and 6000 cps. (Formulation 1)

### EXAMPLE 4

Formulation 2 was prepared to contain a mixture of di(hydrogenated) tallow ammonium di(hydrogenated) tallow maleamate and di(hydrogenated) tallow maleamic acid esentially according to the procedure described above in Example 3.

### EXAMPLE 5

### Preparation of an Anti-dandruff/Conditioning Shampoo

Water (70.0g) was first added into a suitable vessel equipped with agitation, heating and cooling capabilities. While the water was agitated and heated slowly, tetrasodium EDTA (0.2g), STEPANOL® AM-V, (ammonium lauryl sulfate, 2g), and NINOL® 40 CO (cocomide diethanolamine) were added. At about 60°C, a 70:30 mixture of N, N-di(hydrogenated tallow) phthalamic acid and N,N-di(hydrogenated tallow) phthalamic acid N,N-di(hydrogenated tallow) ammonium salt (5g), followed by silicone DC 200 (dimethicone 0.2g) and ZPT (zinc pyrithione, 48% dispersion, 0.6g) were added to the mixture. The mixture was heated to 70-75°C and emulsified for 20-30 minutes at high speed while maintaining the temperature between 70-75°C. The mixture was slowly cooled with agitation set at medium speed. When the temperature of the mixture cooled to about 45°C, 1,3,5,5-tetramethyl hydantoin (0.1g) and ammonium chloride (0.1g) were added. The pH was checked and adjusted as necessary with ammonium hydroxide or citric acid to a value between about 4.5 and 6.5. The viscosity was checked and adjusted as necessary with ammonium chloride to a value between 2000 and 6000 cps. (Formulation 3)

Formulation 4 was prepared to contain a mixture of di(hydrogenated) tallow ammonium di(hydrogenated) tallow maleamate and di(hydrogenated) tallow maleamic acid essentially according to the procedure set forth in Example 5.

### EXAMPLE 6

### Preparation of a conditioning shampoo

Water (108.0 g) was first added into a suitable vessel equipped with agitation, heating, and cooling capabilities. To the agitated water was added α-sulfo-methyl laurate (81.0 g), a 70:30 mixture of N,N-di(hydro-genated tallow) phthalamic acid and N,N-di(hydrogenated tallow) phthalamic acid N,N-di(hydrogenated tallow) ammonium salt (10g.0), and silicone DC200 (12,500 cps, 1.0g). The mixture was heated to about 90°C and was mixed for an additional 10-15 minutes. The temperature of the mixture was slowly cooled and the pH adjusted to about 4.5 to 6.0 with sulfuric acid and/or sodium hydroxide. The mixture was then cooled to room temperature.

### EXAMPLE 7

### Preparation of a conditioning shampoo

Water (163.0g) was first added into a suitable vessel equipped with agitation, heating and cooling capabilities. To the water was added a mixture of C₈₋₁₀ polyethoxy phosphate. The pH was subsequently adjusted to 1.5 to 2.0, if necessary, with sulfuric acid (50%). The mixture was agitated and heated to about 90°C. At about 90°C, di(hydrogenated tallow) amine (6.0g) and silicone DC 200 (12,500cps [12.5 Pa. sec], 1.0g) were added to the mixture. The resulting mixture was then agitated for 20 minutes at 200°F (93.3°C), after which it was cooled to about 90°F (32.2°C). The pH was then adjusted to 4.5 to 6.0 with ammonium hydroxide (28%) and the mixture cooled to room temperature.

### EXAMPLE 8

### Preparation of a conditioning shampoo

Water (89.0 g) was first entered into a suitable vessel equipped with agitation, heating, and cooling capabilities. To the agitated water was added sodium lauryl sulfate (100.0 g), a 70:30 mixture of N,N-di(hydrogenated tallow) phthalamic acid and N,N-di(hydrogenated tallow) phthalamic acid N,N-di(hydrogenated tallow) ammonium salt (10g), and silicone DC200 (12,500 cps, 1.0g). The mixture was heated to about 90°C and was mixed for an additional 10-15 minutes. The temperature of the mixture was slowly cooled and the pH adjusted to about 4.5 to 6.0 with citric acid (50%) and/or sodium hydroxide. The mixture was then cooled to room temperature.

### EXAMPLE 9

### Preparation of a conditioning shampoo

Water (113.0 g) was first added into a suitable vessel equipped with agitation, heating, and cooling capabilities. To the agitated water was added sodium C₁₇₋₁₉ α-olefin sulfonate (38%, 80.0 g), a 70:30 mixture of N,N-di(hydrogenated tallow) amine (6.0g), and silicone DC 200 (1.0g). The mixture was heated to about 90°C and mixed for about an additional 20 minutes. The temperature of the mixture was slowly cooled and the pH adjusted to about 4.5 to 6.0 with sulfuric acid (50%) and/or sodium hydroxide (50%). The mixture was then cooled to room temperature.

### EXAMPLE 10

### Preparation of a conditioning shampoo

Water (117.0 g) was first added into a suitable vessel equipped with agitation, heating, and cooling capabilities. To the agitated water was added sodium dodecylbenzene sulfonate (75.0g), a 70:30 mixture of N,N-di(hydrogenated tallow) amine (6.0g), and silicone DC200 (2.0g). The mixture was heated to about 90°C and was mixed for about an additional 20 minutes. The temperature of the mixture was slowly cooled and the pH adjusted to about 4.5 to 6.0 with sulfuric acid (50%) and/or sodium hydroxide. The mixture was then cooled to roomtemp.

## Claims

1. A method of making a shampoo comprising an alkyl sulfate surfactant, a silicone conditioning agent and a quaternary ammonium salt characterized in that it includes the step of forming a composition of silicone adhered to particles of a suspending agent by heating to a temperature of at least 70°C
(a) a shampoo base comprising an alkyl sulfate surfactant of the formula: 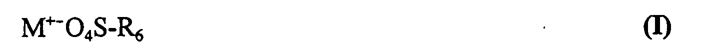 where
R₆ is alkyl having from 10 to 16 carbon atoms; and
M is sodium, potassium, or N₇R₈R₉R₁₀
where R₇R₈R₉R₁₀ are the same or different and represent hydrogen or straight or branched chain alkyl groups having 1 to 8 carbon atoms; (b) a silicone oil; and (c) a di-(C₈ to C₄₀ alkyl) quaternary ammonium salt of the formula: 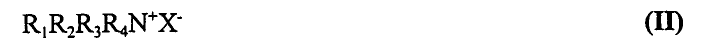 where R₁ and R₂ are the same or different and represent alkyl having 8 to 40 carbon atoms;
R₃ and R₄ are hydrogen; and
X is halide, acetate, nitrate, hydroxide, phosphate;
alkyl sulfate where the alkyl portion has 1-22 carbon atoms;
mono- or di-alkyl phosphate where each alkyl portion has 1-22 carbon atoms;
an anion of the formula
R₅-CO⁻₂
where R₅ is straight or branched chain alkyl having 1-22 carbon atoms; or 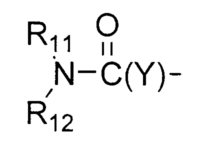 where R₁₁ and R₁₂ are the same or different and represent alkyl or alkenyl having 1-40 carbon atoms; and
Y is ortho, para or meta substituted phenylene, or alkylene or alkenylene having 0-6 carbon atoms, or cycloalkylene having 5-7 carbon atoms.

2. A method according to claim 1, characterised in that the di- (C₈ to C₄₀ alkyl) quaternary ammonium salt is a di(hydrogenated) tallow ammonium salt.

3. A composition comprising a network of silicone oil conditioning agent droplets adhered to a suspending agent which is the reaction product of a di-(C₈ to C₄₀ alkyl) quaternary ammonium salt of the formula (II) set out in claim 1 and an alkyl sulfate surfactant of the formula (I) set out in claim 1.

4. A composition according to claim 3 or claim 4, characterized in that R₁ and R₂ are alkyl groups derived from tallow.

5. A composition according to any one of claims 3 to 5, characterized in that R₆ is lauryl.

6. A composition according to any one of claims 3 to 5 comprising a network of particles of di-(hydrogenated) tallow ammonium lauryl sulfate adhered to droplets of a silicone oil conditioning agent.

7. A composition according to any one of claims 3 to 6, characterized in that it is formulated as a conditioning shampoo.

8. A shampoo comprising an alkyl sulfate surfactant and a network of an effective conditioning amount of silicone oil conditioning agent droplets adhered to a suspending agent which is the reaction product of a di-(C₈ to C₄₀ alkyl alkyl) quaternary ammonium salt of the formula (II) set out in claim 1 and an alkyl sulfate surfactant of the formula (I) set out in claim 1.

9. A shampoo according to claim 8, characterized in that R₆ is lauryl.

10. A shampoo according to claim 8 or claim 9, characterized in that R₁ and R₂ are alkyl groups derived from tallow.

11. A conditioning shampoo comprising a silicone oil conditioning agent stably suspended in a network of silicone oil adhered to a suspending agent, the shampoo being prepared by a process comprising the steps of (a) mixing an alkyl ammonium salt of the formula R₁R₂NH₂⁺X⁻, where X is as defined in claim 1 and R₁ and R₂ are the same or different and represent straight or branched alkyl groups having from 14 to 20 carbon atoms, and an excess of an alkyl sulfate surfactant in the shampoo, the alkyl sulfate surfactant being of the formula:
M^{+ -}O₄S-R₆ (I)
where M and R₆ are as defined above; and (b) heating the shampoo above 70°C.

## Patentansprüche

1. Verfahren zur Herstellung eines Haarwaschmittels, das ein oberflächenaktives Alkylsulfat, ein Silikon-Konditioniermittel und ein quaternäres Ammoniumsalz enthält, dadurch gekennzeichnet, daß es die Stufe der Bildung einer Zusammensetzung von Silikon in Haftung an Suspendiermittelteilchen umfaßt, durch Erhitzen auf eine Temperatur von wenigstens 70°C
(a) einer Haarwaschmittelbasis enthaltend ein oberflächenaktives Alkylsulfat der Formel
M^{+ -}O₄S-R₆ (I)
worin R₆ ein Alkyl mit 10 bis 16 Kohlenstoffatomen ist und M Natrium, Kalium oder NR₇R₈R₉R₁₀
ist, worin R₇, R₈, R₉ und R₁₀ gleich oder verschieden sind und Wasserstoff oder gerade oder verzweigtkettige Alkylgruppen mit 1 bis 8 Kohlenstoffatomen bedeuten;
(b) eines Silikonöls; und
(c) eines di(C₈- bis C₄₀-alkyl)quaternären Ammoniumsalzes der Formel
R₁R₂R₃R₄N⁺ X⁻ (II)
worin R₁ und R₂ gleich oder verschieden sind und Alkyl mit 8 bis 40 Kohlenstoffatomen bedeuten, R₃ und R₄ Wasserstoff sind, und X Halogenid, Acetat, Nitrat, Hydroxid oder Phosphat ist;
von Alkylsulfat, in dem der Alkylteil 1 bis 22 Kohlenstoffatome hat;
von Mono- oder Dialkylphosphat, in dem jeder Alkylteil 1 bis 22 Kohlenstoffatome hat;
eines Anions der Formel R₅-CO₂⁻, in der R₅ ein gerades oder verzweigtkettiges Alkyl mit 1 bis 22 Kohlenstoffatomen ist; oder von 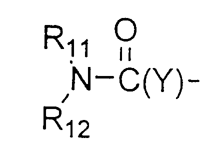 worin R₁₁ und R₁₂ gleich oder verschieden sind und Alkyl oder Alkenyl mit 1 bis 40 Kohlenstoffatomen bedeuten und Y ortho-, para- oder meta-substituiertes Phenylen oder Alkylen oder Alkenylen mit 0 bis 6 Kohlenstoffatomen oder Cycloalkylen mit 5 bis 7 Kohlenstoffatomen ist.

2. Verfahren nach Anspruch 1, dadurch gekennzeichnet, daß das di(C₈- bis C₄₀-alkyl)quaternäre Ammoniumsalz ein di(hydriertes) Talgammoniumsalz ist.

3. Zusammensetzung mit einem Netzwerk von Silikonöl-Konditioniermitteltröpfchen, die an einem Suspendiermittel haften, das das Reaktionsprodukt eines di(C₈- bis C₄₀alkyl)quaternären Ammoniumsalzes der in Anspruch 1 angegebenen Formel (II) mit einem oberflächenaktiven Alkylsulfat der in Anspruch 1 angegebenen Formel (I) ist.

4. Zusammensetzung nach Anspruch 3, dadurch gekennzeichnet, daß R₁ und R₂ aus Talg abgeleitete Alkylgruppen sind.

5. Zusammensetzung nach Anspruch 3 oder 4, dadurch gekennzeichnet, daß R₆ Lauryl ist.

6. Zusammensetzung nach einem der Ansprüche 3 bis 5 mit einem Netzwerk von Teilchen aus di(hydriertem) Talg-Ammoniumlaurylsulfat, die an Tröpfchen aus einem Silikonöl-Konditioniermittel haften.

7. Zusammensetzung nach einem der Ansprüche 3 bis 6, dadurch gekennzeichnet, daß sie als ein Konditionier-Haarwaschmittel formuliert ist.

8. Haarwaschmittel mit einem oberflächenaktiven Alkylsulfat und einem Netzwerk aus einer wirksamen Konditioniermenge von Silikonöl-Konditioniermitteltröpfchen, die an einem Suspendiermittel haften, welches das Reaktionsprodukt eines di(C₈- bis C₄₀-alkyl-alkyl)quaternären Ammoniumsalzes der in Anspruch 1 angegebenen Formel (II) mit einem oberflächenaktiven Alkylsulfat der in Anspruch 1 angegebenen Formel (I) ist.

9. Haarwaschmittel nach Anspruch 8, dadurch gekennzeichnet, daß R₆ Lauryl ist.

10. Haarwaschmittel nach Anspruch 8 oder Anspruch 9, dadurch gekennzeichnet, daß R₁ und R₂ Alkylgruppen sind, die sich von Talg ableiten.

11. Konditionier-Haarwaschmittel mit einem Silikonöl-Konditioniermittel, das in einem Netzwerk von Silikonöl in Haftung an einem Suspendiermittel stabil suspendiert ist, wobei das Haarwaschmittel durch ein Verfahren hergestellt ist, bei dem man
(a) ein Alkylammoniumsalz der Formel R₁R₂NH₂⁺X⁻, in der X wie in Anspruch 1 definiert ist und R₁ und R₂ gleich oder verschieden sind und gerade und verzweigte Alkylgruppen mit 14 bis 20 Kohlenstoffatomen bedeuten, und ein Überschuß eines oberflächenaktiven Alkylsulfats in dem Haarwaschmittel mischt, wobei das oberflächenaktive Alkylsulfat die Formel
M^{+ -}O₄S-R₆ (I)
hat, worin M und R₆ wie oben definiert sind, und
(b) das Haarwaschmittel über 70°C erhitzt.

## Revendications

1. Procédé pour préparer un shampooing comprenant un tensioactif alkylsulfate, un agent traitant siliconé et un sel d'ammonium quaternaire, caractérisé en ce qu'il comprend l'étape de formation d'une composition de silicone adhérant à des particules d'un agent de mise en suspension par chauffage à une température d'au moins 70°C :
(a) d'une base de shampooing comprenant un tensioactif alkylsulfate de formule :
M^{+ -}O₄S-R₆ (I)
dans laquelle R₆ est un alkyle ayant de 10 à 16 atomes de carbone ; et M est le sodium, le potassium ou N₇R₃R₉R₁₀, où R₇, R₈, R₉ et R₁₀ peuvent être identiques ou différents et représentent l'hydrogène ou des groupes alkyle à chaîne droite ou ramifiée ayant de 1 à 8 atomes de carbone ;
(b) d'une huile de silicone ; et
(c) d'un sel de di(alkyl en C₈ à C₄₀)ammonium quaternaire de formule :
R₁R₂R₃R₄N⁺X⁻ (II)
dans laquelle R₁ et R₂ sont identiques ou différents et représentent des groupes alkyle ayant de 8 à 40 atomes de carbone ;
R₃ et R₄ sont l'hydrogène ; et
X est un radical halogénure, acétate, nitrate, hydroxyde, phosphate ; alkylsulfate où le fragment alkyle a de 1 à 22 atomes de carbone, mono- ou dialkylphosphate où chaque fragment alkyle a de 1 à 22 atomes de carbone ; un anion de formule :
R₅-CO⁻₂
dans laquelle R₅ est un groupe alkyle à chaîne droite ou ramifiée ayant de 1 à 22 atomes de carbone ; ou 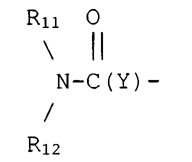 dans laquelle R₁₁ et R₁₂ sont identiques ou différents et représente un groupe alkyle ou alcényle ayant de 1 à 40 atomes de carbone ; et
Y est un radical phénylène à substitution ortho, para ou méta ou alkylène ou encore alcénylène ayant de 0 à 6 atomes de carbone, ou bien cycloalkylène ayant de 5 à 7 atomes de carbone.

2. Procédé selon la revendication 1, caractérisé en ce que le sel de di(alkyl en C₈ à C₄₀)ammonium quaternaire est un sel de di(suif-yl hydrogéné)ammonium.

3. Composition comprenant un réseau de gouttelettes d'agents traitants de type huile de silicone adhérant à un agent de mise en suspension qui est le produit de la réaction d'un sel de di(alkyl en C₈ à C₄₀)ammonium quaternaire de formule (II) décrit dans la revendication 1 et d'un tensioactif alkylsulfate de formule (I) décrit dans la revendication 1.

4. Composition selon la revendication 3 ou 4, caractérisée en ce que R₁ et R₂ sont des groupes alkyle dérivant du suif.

5. Composition selon l'une quelconque des revendications 3 à 5, caractérisée en ce que R₆ est le radical lauryle.

6. Composition selon l'une quelconque des revendications 3 à 5, comprenant un réseau de particules de laurylsulfate de di(suif-yl hydrogéné)ammonium adhérant à des gouttelettes d'un agent traitant de type huile de silicone.

7. Composition selon l'une quelconque des revendications 3 à 6, caractérisée en ce qu'elle est formulée dans un shampooing traitant.

8. Shampooing comprenant un tensioactif alkylsulfate et un réseau d'une quantité nécessaire à un traitement efficace de gouttelettes d'agent traitant de type huile de silicone adhérant à un agent de mise en suspension qui est le produit de la réaction d'un sel de di(alkyl en C₉ à C₄₀)ammonium quaternaire de formule (II) décrit dans la revendication 1 et d'un tensioactif alkylsulfate de formule (I) décrit dans la revendication 1.

9. Shampooing selon la revendication 8, caractérisé en ce que R₆ est le radical lauryle.

10. Shampooing selon la revendication 8 ou 9, caractérisé en ce que R₁ et R₂ sont des groupes alkyle dérivant du suif.

11. Shampooing traitant comprenant un agent traitant de type huile de silicone en suspension stable dans un réseau d'huile de silicone adhérant à un agent de mise en suspension, le shampooing étant préparé par un procédé comprenant les étapes consistant à :
(a) mélanger un sel d'alkylammonium de formule R₁R₂NH₂⁺X⁻ dans laquelle X est tel que défini dans la revendication 1 et R₁ et R₂ sont identiques ou différents et représentent des groupes alkyle à chaîne droite ou ramifiée ayant de 14 à 20 atomes de carbone, avec un excès d'un tensioactif alkylsulfate dans le shampooing, le tensioactif alkylsulfate répondant à la formule :
M^{+ -}O₄S-R₆ (I)
dans laquelle M et R₆ sont tels que définis ci-dessus ; et
(b) chauffer le shampooing au-delà de 70°C.
